(19) Europäisches Patentamt
European Patent Office
Office européen des brevets

(11) **EP 1 343 391 B1**

(12) **EUROPÄISCHE PATENTSCHRIFT**

(45) Veröffentlichungstag und Bekanntmachung des Hinweises auf die Patenterteilung:
**11.10.2006 Patentblatt 2006/41**

(51) Int Cl.:
*A23L 3/015* (2006.01)   *A23B 4/00* (2006.01)
*A61L 11/00* (2006.01)   *A61L 2/02* (2006.01)
*C07K 1/113* (2006.01)

(21) Anmeldenummer: **01271155.2**

(22) Anmeldetag: **19.12.2001**

(86) Internationale Anmeldenummer:
**PCT/EP2001/015027**

(87) Internationale Veröffentlichungsnummer:
**WO 2002/049460 (27.06.2002 Gazette 2002/26)**

(54) **VERFAHREN ZUR GEZIELTEN VERÄNDERUNG DER PROTEINSTRUKTUR VON PRIONEN PRPsc**

METHOD FOR MODIFYING THE PROTEIN STRUCTURE OF PRIONS PRPsc IN A TARGETED MANNER

PROCEDE DE MODIFICATION CIBLEE DE LA STRUCTURE PROTEIQUE DE PRIONS PRPsc

(84) Benannte Vertragsstaaten:
**AT BE CH CY DE DK ES FI FR GB GR IE IT LI LU MC NL PT SE TR**

(30) Priorität: **20.12.2000 DE 10063667**
**03.12.2001 DE 10159248**

(43) Veröffentlichungstag der Anmeldung:
**17.09.2003 Patentblatt 2003/38**

(73) Patentinhaber: **Kortschack, Fritz**
**14089 Berlin (DE)**

(72) Erfinder:
• **KORTSCHACK, Fritz**
**14089 Berlin (DE)**
• **HEINZ, Volker**
**13595 Berlin (DE)**

(74) Vertreter: **Kruspig, Volkmar et al**
**Meissner, Bolte & Partner GbR**
**Postfach 86 06 24**
**81633 München (DE)**

(56) Entgegenhaltungen:
**EP-A- 1 021 958          WO-A-01/09287**
**WO-A-98/26667          WO-A-99/25206**
**DE-A- 10 106 115          DE-U- 20 021 445**
**US-A- 5 213 029          US-A- 6 017 572**
**US-A- 6 086 936**

• **LEHMANN G: "HOCHDRUCKBEHANDLUNG - EINE NEUE LEBENSMITTELTECHNOLOGIE" FLEISCHWIRTSCHAFT, FRANKFURT, DE, Bd. 76, Nr. 10, 1996, Seiten 1004-1005, XP002061566 ISSN: 0015-363X**
• **ZHOU J. : "Pressure denaturation of the Yeast prion protein Ure2" BIOCHEMICAL AND BIOPHYSICAL RESEARCH COMMUNICATIONS, Bd. 287, Nr. 1, September 2001 (2001-09), Seiten 147-152, XP002192684 in der Anmeldung erwähnt**
• **PATENT ABSTRACTS OF JAPAN vol. 017, no. 124 (C-1035), 16. März 1993 (1993-03-16) -& JP 04 304838 A (TOPPAN PRINTING CO LTD), 28. Oktober 1992 (1992-10-28)**
• **PATENT ABSTRACTS OF JAPAN vol. 017, no. 180 (C-1046), 8. April 1993 (1993-04-08) -& JP 04 335873 A (ATSUSHI SUZUKI;OTHERS: 01), 24. November 1992 (1992-11-24)**

**Beschreibung**

Die Erfindung betrifft ein Verfahren zur gezielten Veränderung der Proteinstruktur von Prionen PrP$^{SC}$ oder infektiösen PrP$^{SC}$-Ketten in tierischen, zum Verzehr oder zur Weiterverarbeitung vorgesehenen Rohprodukten, insbesondere Fleisch, Unterhäute, Bindegewebe, Hirn und Knochen und/oder zur Sterilisation von Schlachthausgerätschaften, Operationsbestecken oder dergleichen mit infektiösen PrP$^{SC}$ kontaminierten Gegenständen, gemäß Oberbegriff des Patentanspruchs 1.

Es hat sich durch umfangreiche Untersuchungen gezeigt, daß die Erreger von Scrapie und BSE außerordentlich resistent gegen physikalische und chemische Einflüsse sind. Prionen sind keine Krankheitserreger im klassischen Sinne. Sie unterscheiden sich wesentlich von Bakterien oder Viren. Hitze von 100 Grad Celsius; Chemikalien oder viele Desinfektionsmittel können Ihnen nichts anhaben. Prionen sind auch die Erreger weiterer Krankheiten bei Tieren, wie der Traberkrankheit (Scrapie) bei Schafen, und Menschen, wie dem sehr seltenen Gerstmann-Sträussler-Syndrom. Man nimmt an, dass die Erkrankung vom Tier auf den Menschen übertragen werden kann.

Aus neuen Untersuchungen über die Strukturen des Prionproteins wurden Kenntnisse über die Entstehung von CJD, GSS, BSE, Scrapie und CWD gewonnen. Nach diesen Kenntnissen geht krankheitsdeterminierend das Prion-Protein in eine geänderte dreidimensionale Faltung über, wobei dieses umgefaltete Protein PrP$^{SC}$ der krankheitsauslösende Faktor ist. Kommt nun ein natürliches Prion PrP$^{C}$ in Kontakt mit einem entarteten, gefalteten Protein, verändert es seine Form und regt weitere Prionen zur Umfaltung an. Auf der Oberfläche der Zelle häufen sich dann die infizierten Prionen an und die Nervenzelle stirbt ab.

Zur Bedeutung der BSE-Problematik allein in Deutschland sei auf folgende Fakten verwiesen.

Nach vorliegenden amtlichen Berechnungen wurden im Jahr 2000 in Deutschland über 3,8 Millionen Rinder mit einem durchschnittlichen Schlachtgewicht von 323kg und über 410.000 Kälber mit einem durchschnittlichen Schlachtgewicht von 125kg geschlachtet. Unter den geschlachteten Rindern waren über 630.000 Färsen mit einem durchschnittlichen Schlachtgewicht von 287kg, 1,5 Millionen Kühe mit einem durchschnittlichen Schlachtgewicht von 297 kg und 1,6 Millionen Bullen mit einem durchschnittlichen Schlachtgewicht von etwa 361kg. Weiterhin wurden allein im Jahr 2000 in Deutschland mindestens 42.000 Ochsen im Alter von 2 bis 3 Jahren geschlachtet. Mit Ausnahme von etwa 20.000 Zuchtbullen und etwas mehr als der Hälfte der geschlachteten Kälber werden fast alle männlichen Rinder als Jungbullen im Alter von 18 bis 24 Monaten geschlachtet. Bei den Kälbern muß man im allgemeinen noch nicht mit relevanten Mengen von Infektiosität rechnen. Bei den 18 bis 24 Monaten alten Jungbullen und den selten älteren Färsen ist selbst im Fall einer BSE-Infektion zumindest das Zentralnervengewebe sehr wenig infektiös. Hochinfektiös sind hingegen Hirn und Rückenmark von BSEinfizierten Kühen und Bullen. In der relevanten Altersklasse werden also in Deutschland jährlich etwa 1,566 Millionen Kühe, Ochsen und Bullen geschlachtet.

Bis zum 11.2.2001 wurden rund 209.000 BSE-Tests nach dem Fleischhygienerecht durchgeführt. Zu diesen Tests kommen noch die BSE-Tests bei gefallenen und notgeschlachteten Tieren hinzu. Bei bisher 17 (Stand 28.02. 2001) bestätigten BSE-Fällen bei im normalem Schlachthof oder beim Metzger geschlachteten Rindern muß etwa ungefähr mit 1 hochinfektiösen Rind pro 249.000 / 17 = 14.647 normal geschlachteten Tieren gerechnet werden. Hochgerechnet auf die jährlichen Schlachtzahlen sind dies ungefähr 100, das Abwasser deutscher Schlachthöfe belastende Rinder. Weil bis ins Jahr 2000 Schlachtabfallprodukte einer zunehmenden Anzahl BSE-infizierter Rinder an Rinder und insbesondere Kälber verfüttert wurden, wird der Anteil der infizierten Tiere in den kommenden Jahren wahrscheinlich noch deutlich zunehmen.

Allein in den Schlachthöfen werden durch den Bolzenschuß, das Absetzen und Reinigen der Köpfe und das Aufsägen der Wirbelsäulen pro Rind etwa 10g Hirn und Rückenmark aus dem Schlachtkörper gepreßt oder gerissen. Dieser feine Gewebebrei wird mit Wasser von den Schlachtkörpern und Köpfen gespült und gelangt ins Abwasser. Bei 100 geschlachteten BSE-Rindern wären das 1000g hochinfektiöses Zentralnervengewebe, welches bei Aufnahme über die Nahrung jährlich etwa 5000 Rinder tödlich infizieren könnte. Diese recht große Ausgangsmenge infiziösen Gewebebreis gelangt unsterilisiert in die Kanalisation und aufgrund der hohen Fließgeschwindigkeiten größtenteils in die Kläranlagen. Würde man davon ausgehen, daß der Klärschlamm auf Rinderweiden zu verteilen ist, dann müßte mit einer noch erheblicheren Gefährdung der Rinder gerechnet werden, weil diese mit dem Gras auch täglich etwa 5kg Erde aufnehmen. Experimente haben gezeigt, daß der vollständige Abbau der Infektiosität im Boden einige bis viele Jahre dauern kann. Daher muß man damit rechnen, daß die Infektiosität teilweise in humusbildende Strukturen fest eingebunden und darin sehr stabil gelagert werden kann. Auf jeden Fall kann es während der Zeit des langsamen Abbaus, beispielsweise durch Bodenbakterien, dazu kommen, daß die Prionen über die Nahrungsketten des Bodens von der Aufnahme durch Kleinstlebewesen bis in kleine Säugetiere gelangen, die dann infiziert werden könnten.

Aus den vorgenannten Darlegungen ergibt sich die klare Relevanz dafür, daß medizinische Geräte und tierische Rohstoffe für Nahrungsmittel, Medikamente und Kosmetika so zu sterilisieren sind, daß auch infektiöse Prionproteine inaktiviert werden. Eine Inaktivierung ist nur dann dauerhaft wirksam, wenn sie wirklich vollständig war, wie experimentelle Untersuchungen zeigen.

Aus der Lebensmitteltechnologie ist seit Anfang der 80er Jahre die Hochdruckbehandlung für Nahrungsmittel bekannt, einerseits um diese zu entkeimen und andererseits um Effekte zu erzielen, die wie bei der Nahrungszubereitung durch Kochen mit hoher Temperatur erreicht werden. Hierbei kommt es zu einer Denaturierung von Eiweiß, zur Inaktivierung von Enzymen und zur Gelatinierung der Stärke neben der Abtötung von Mikroorganismen. Durch die Hochdruckbehandlung werden große Moleküle beeinflußt, während kleinere, wie Aminosäuren, Vitamine oder Geschmacksstoffe erhalten bleiben.

Die Hochdruckbehandlung selbst stellt einen nichtthermischen Prozeß dar, der mit hydrostatischen Drücken im Bereich von mehreren 1000 bar arbeitet. Nach der Behandlung finden elastische Produkte beim Entspannen wieder in ihre ursprüngliche Form und Größe zurück. Bekannt ist es, in flexible Folien verpackte Lebensmittel in Chargiergestelle oder -körper einzulegen, die dann in eine mit Wasser als Druckträger gefüllten Behälter eingeführt werden. Der Druckbehälter wird nach dem Beladen geschlossen und der Druck aufgebaut, indem ein Kolben in den Druckbehälter eingefahren wird, bis der voreingestellte Druck erreicht ist. Grundsätzlich gilt, daß der Druck der wichtigste Parameter ist, wobei Zeit und Temperatur nur eine untergeordnete Rolle spielen, da die notwendige Behandlungsenergie über den Druck in das System eingebracht wird.

Durch den Druck entsteht Kompressionswärme deren Verlauf von der Zusammensetzung des Behandlungsgutes abhängig ist.

Produkte mit einem hohen Fettanteil erreichen durch die Kompressionswärme ein höheres Temperaturniveau als reines Wasser. Der druckabhängige Temperaturanstieg kann zur gezielten Behandlung des Gutes eingesetzt werden. Nach Beendigung der Druckbehandlung sinkt die Temperatur, auch des Behandlungsgutes, zeitgleich wieder auf das Ausgangstemperaturniveau zurück.

Grundlagen zur Hochdruckbehandlung im Bereich der Lebensmitteltechnologie sind beispielsweise in Lehmann, G: "Hochdruckbehandlung - eine neue Lebensmitteltechnologie"; Fleischwirtschaft, Frankfurt, Bd. 76, Nr. 10, 1996, Seiten 1004 - 1005, abgehandelt.

Die Behandlung von Lebensmitteln mittels Hochdruck auch unter dem Einhalten verschiedener Behandlungszyklen ist beispielsweise in den US-Patenten 6,086,936 oder 6,017 572 erläutert.

Ergänzend ist bezüglich des Standes der Technik auf eine thermische Behandlung von tierischen Stoffen im Zusammenhang mit BSE-Erkrankungen nach EP 1 021 958 A1 aufmerksam zu machen.

Letztendlich zeigt die gattungsbildende WO 01/09287 A2 ein Druckbehandlungsverfahren von Zellen zur Veränderung der Replikationsfähigkeit und/oder der Proteinstruktur. Das dortige Verfahren ist dadurch gekennzeichnet, daß die Zellen und/oder das Gewebe und/oder die in oder mit diesen Zellen vorliegenden Mikroorganismen Drücken von über 100 MPa in einem bei diesen Drücken flüssigen Medium ausgesetzt werden.

Aus dem Vorgenannten ist es daher Aufgabe der Erfindung, ein weiterentwickeltes Verfahren zur gezielten Veränderung der Proteinstruktur von Prionen $PrP^{SC}$ in tierischen, zum Verzehr oder zur Weiterverarbeitung vorgesehenen Rohprodukten, aber auch zur Sterilisation von medizinischen Geräten, Schlachthausgerätschaften oder dergleichen anzugeben, wobei die Behandlung selbst die übrigen, gewünschten Eigenschaften der Rohprodukte nicht oder nur in geringem Maße beeinflussen soll bzw. der insgesamt anfallende Behandlungsaufwand reduziert ist.

Die Lösung der Aufgabe der Erfindung erfolgt mit einem Verfahren gemäß den Merkmalen des Patentanspruchs 1.

Es wird die Erkenntnis genutzt, daß durch eine Hochdruckbehandlung, welche an sich bekannt ist, die Proteinstruktur von Prionen so verändert werden kann, daß eine Deaktivierung dieser zur Vermeidung von BSE, CJD, GSS, CWD oder Scrapie eintritt. Bezüglich der tatsächlichen überraschenden Wirkungen sei auf den nachveröffentlichten Artikel in "Biochemical and Biophysical Research Communications" 287, 147 bis 152 (2001): Pressure Denaturation of the Yeast Prion Protein Ure2, dort insbesondere Seite 149, linke Spalte Mitte, verwiesen.

Es wird über ein Übertragungsmedium das jeweilige flüssigkeitsumspülte oder in einer feuchten Umgebung befindliche Rohprodukt einer intensiven, von allen Seiten wirkenden Hochdruckbehandlung unterzogen, wobei auch eine spezifizierte Temperaturbehandlung erfolgt.

Bei der Behandlung wird das Rohprodukt verdichtet, gasgefüllte Hohlräume werden entfernt und entartete gefaltete Proteine werden deaktiviert, so daß die Gefahr ausgeschlossen ist, daß beim Inkontaktkommen des entarteten gefalteten Proteins mit einem Prion $PrP^{C}$ weitere Prionen zur Umfaltung angeregt werden.

Bei einem Ausführungsbeispiel der Erfindung wurden verschiedene Rohprodukte mit thermoplastischem Material umhüllt, in einen Druckbehälter überführt und dort auf ca. 80° C erhitzt. Es erfolgte dann ein Druckaufbau bis hin zu einem Maximaldruck von etwa 8000 bar. Durch die Kompressionswärme erfolgte eine Temperaturerhöhung auf ca. 110°C. Die Behandlung dauerte insgesamt 15 Minuten. Mit dem Abbau des Drucks reduzierte sich die Temperatur wieder auf den Ausgangswert von 80° C.

Es konnte dann festgestellt werden, daß aufgrund der Behandlung eine BSE-Infektivität nicht mehr gegeben war.

Der Nachweis der Prioneninaktivierung wurde mittels eines biochemischen Testkits zur Feststellung der Prionenresistenz gegenüber Proteinase K durchgeführt. Bei dieser Methode wird davon ausgegangen, daß nur die infektiöse Form des Prion Proteins die Verdauerung durch das Enzympräparat übersteht. Die Detektion der verbleibenden Prionen erfolgt durch speziell entwickelte Antikörper und Nachweisverfahren wie z.B. Western Blot oder LIA.

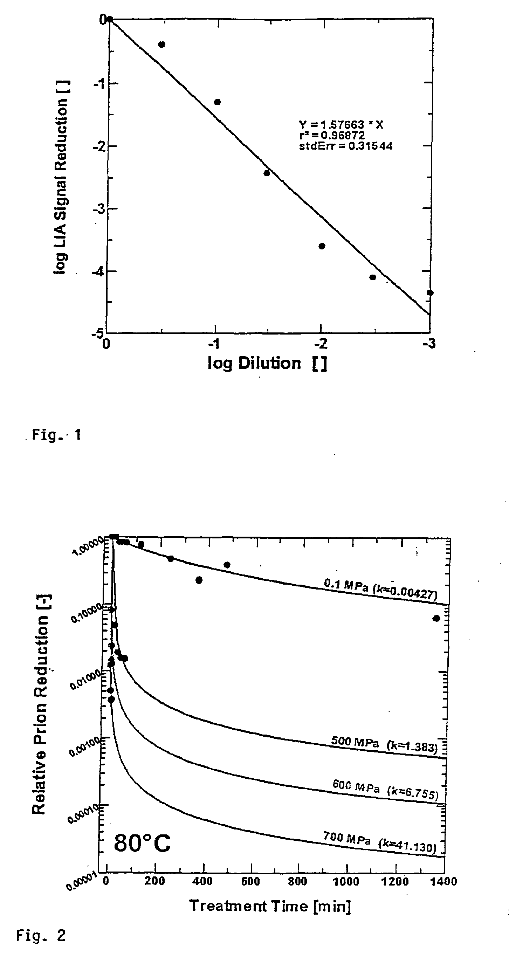

Zur Quantifizierung der Inaktivierungsergebnisse mittels LIA-Test müssen Vergleichstests vorgenommen werden. Hierzu wurde eine positiv beurteilte Probe (Gehirnhomogenisat einer BSE-Kuh) mit Material von negativen Proben in definierten Stufen verdünnt, wodurch sich die Konzentration der Prionen im gleichen Verhältnis reduziert. Diese Reduktion wird üblicherweise logarithmisch aufgetragen. Die Normierung der bei den Verdünnungen erhaltenen LIA-Werte erfolgte mittels Division durch den jeweiligen LIA-Anfangswert $LIA/LIA_0$. Die Fig. 1 und die angegebene Gleichung dient für die Inaktivierungsversuche als Kalibrierung. Im logarithmischen Maßstab ergibt sich ein linearer Zusammenhang. Mit dieser Gleichung wurden nun die LIA-Werte vor der kinetischen Analyse in Verdünnungsfaktoren umgerechnet (-1=1:10; -2=1:100; -3=1:1000 etc).

Die Ermittlung des Druckeffekts auf die Prioneninaktivierung erfolgte durch die Analyse der Reduktion des LIA-Parameters im zeitlichen Verlauf der Einwirkung konstanten hydrostatischen Drucks bei konstanter Temperatur auf in Kunststoffampullen verpacktes Homogenisat von BSE-positiv getesteten Rinderhirn. Derartige Untersuchungen wurden auf verschiedenen Druck- und Temperaturniveaus und bei ausgewählten zeitlichen Stützstellen durchgeführt.

Die Auftragung der in Verdünnungsfaktoren umgerechneten LIA-Werte im halblogarithmischen Maßstab ergab die für die weitergehende kinetische Analyse benötigten Inaktivierungskurven. Am Beispiel des Temperaturniveaus 80°C ist der zeitliche Inaktivierungsverlauf bei Umgebungsdruck (0,1 MPa), 500 MPa, 600 MPa und 700 MPa in der Fig. 2 dargestellt. Typischerweise werden auf allen Druckniveaus stark gekrümmte Kurven mit stetig abnehmender Steilheit erhalten.

Die zur Konkavität führende Abnahme der Inaktivierungsgeschwindigkeit kann auf druck- bzw. thermoresistente Subpopulationen der Prionengesamtheit zurückgeführt werden.

Die in der Fig. 2 gezeigte Extrapolation der experimentellen Ergebnisse wurde mittels einer kinetischen Analyse durchgeführt, die auf der Annahme einer Proportionalität zwischen der Inaktivierungsgeschwindigkeit dC/dt und der exponentiell korrigierten Prionenkonzentration $C^n$ beruht. n wird bei diesem Konzept als Reaktionsordnung bezeichnet. Nach Einführung des Proportionalitätsfaktors k (=Geschwindigkeitskonstante) ergibt sich folgende Differentialgleichung:

$$\frac{dC}{dt} = -k \cdot C^n \qquad \text{Gleichung 1}$$

In integrierter Form ergibt sich folgendes Zeitgesetz:

$$\left(\frac{C}{C_0}\right) = \left(1 + k \cdot t \cdot (n-1)\right)^{\frac{1}{1-n}} \qquad \text{Gleichung 2}$$

Die linke Seite dieser Gleichung entspricht der relativen Abnahme der Prionenkonzentration ausgedrückt in Verdünnungsfaktoren gemäß der oben beschriebenen Kalibrierung. Die Geschwindigkeitskonstante k kann regressiv ermittelt werden.

Die Bestimmung der Reaktionsordnung erfolgte durch die Minimierung des aufsummierten Standardfehlers aller verfügbaren Inaktivierungsdaten auf verschiedenen Temperatur- und Druckniveaus.

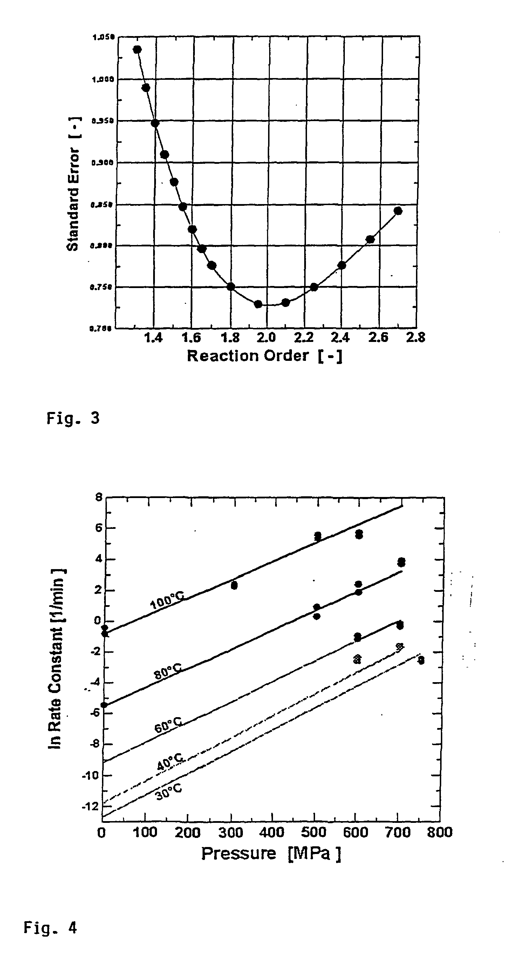

Wie aus Fig. 3 ersichtlich ist, durchläuft die Standardfehlerfunktion bei einer Reaktionsordnung von n=2 ein Minimum.

Somit können unter einheitlicher Verwendung der Reaktionsordnung von n=2,0 die Parameter des Zeitgesetzes der Inaktivierungsreaktion zur Beschreibung des Druck- und Temperatureinflusses auf die Geschwindigkeitskonstante k reduziert werden.

Für die vollständige Beschreibung muß für k eine Funktion der Temperatur und des Drucks gefunden werden. In Fig. 4 sind die aus den Regressionsanalysen der Inaktivierungskinetiken ermittelten Geschwindigkeitskonstanten k dargestellt (Punkte). Aus der logarithmischen Auftragung von k gegen den Behandlungsdruck kann ein linearer Zusammenhang abgeleitet werden. Üblicherweise wird dies folgendermaßen formuliert (siehe z.B. Morild, E. (1981) The Theory of Pressure Effects on Enzymes. Advances in Protein Chemistry 34: 93-167):

$$\ln(k) = k_0 + \left(\frac{-\Delta V^{\#}}{R \cdot T}\right) \cdot p \qquad \text{Gleichung 3}$$

Als charakteristischer Parameter tritt in dieser Gleichung das sogenannte Aktivierungsvolumen $\Delta V^{\#}$, das meist in [mL/mol] angegeben ist. R ist die molare Gaskonstante: 8,314 J/(mol K). $k_0$ kann näherungsweise als die Geschwindigkeitskonstante bei Umgebungsdruck (0,1 MPa) auf dem jeweiligen Temperaturniveau betrachtet werden. Die Approximierung der experimentellen Ergebnisse erfolgte mittels eins Polynoms 2. Ordnung. Die resulierende Gesamtgleichung lautet daher:

$$\ln(k) = AO + A1 \cdot T + A2 \cdot T^2 + \left(\frac{-\Delta V^{\#}}{R \cdot T}\right) \cdot p \qquad \text{Gleichung 4}$$

Nach regressiver Ermittlung der Parameter ergibt sich:

$$\ln(k) = 86,05 - 0,728 \cdot T + 0,00133 \cdot T^2 + \left(\frac{36,79}{R \cdot T}\right) \cdot p \qquad \text{Gleichung 5}$$

Für die Temperaturniveaus: 30, 40, 60, 80 und 100°C ist die Funktion in der Fig. 4 grafisch dargestellt.
Auf der Grundlage der bisher verfügbaren experimentellen Ergebnisse kann somit als charakteristische Größe der Druckabhängigkeit der Inaktivierungkinetik ein Aktivierungsvolumen von $\Delta V^{\#}$ = -36,79 $\pm$ 2,88 mL/mol bei einem Signifikanzniveau von 0,95 angegeben werden.
Zur Berechnung der zu erwartenden Prioneninaktivierung (angegeben in Zehnerpotenzen) für die ermittelte Reaktionsordnung von n=2 können zusammenfassend folgende Funktionen angegeben werden:

a)
Für den Fall einer Behandlung der Dauer t (in Minuten) bei gleichbleibendem Temperatur- und Druckniveau, wobei die Kompressions- und Dekompressionszeiten gegenüber der Einwirkungszeit vernachlässigt werden:

$$\log\left(\frac{C_t}{C_0}\right) = \log\left(\frac{1}{1 + k \cdot t}\right) \qquad \text{Gleichung 6}$$

Die für das jeweilige Temperatur und Druckniveau gültige Geschwindigkeitskonstante kann aus Gleichung 8 errechnet werden.

b)
Die bis zum Zeitpunkt t (in Minuten) erreichte Inaktivierung für den Fall, daß Änderungen von Druck und Temperatur während der Behandlungszeit berücksichtigt werden müssen:

$$\log\left(\frac{C_t}{C_0}\right) = \log\left(\frac{1}{1 + \int_0^t k \, dt}\right) \qquad \text{Gleichung 7}$$

Da die Geschwindigkeitskonstante k eine Funktion der zeitlich variablen Größen Druck und Temperatur ist, müssen in Gleichung 8 Temperatur und Druck als die bei der Behandlung verwendeten Zeitfunktionen eingesetzt und wie in Gleichung 7 dargestellt integriert werden.

$$\ln(k) = 86,05 - 0,728 \cdot T + 0,00133 \cdot T^2 + \left(\frac{36,79}{R \cdot T}\right) \cdot p \qquad \text{Gleichung 8}$$

mit:

$C_t/C_0$: Reduktion der Prionenkonzentration
k: Geschwindigkeitskonstante [1/min]
R: molare Gaskonstante: 8,314 J/(mol K)
p: Druck [MPa]
T: Temperatur [K]

In der Literatur sind Richtwerte für die erforderliche Behandlungsintensität zur sicheren Hitzeinaktivierung CJD infizierter Materialien angegeben (siehe z.B. Casolari, A. (1998) Heat Resistance of Prions and Food Processing, Food Microbiology 15: 59-63):

• 18 Minuten bei 134°C laut Department of Health and Social Security (DHSS)
• 60 Minuten bei 132°C laut American Neurobiological Association (ANA)

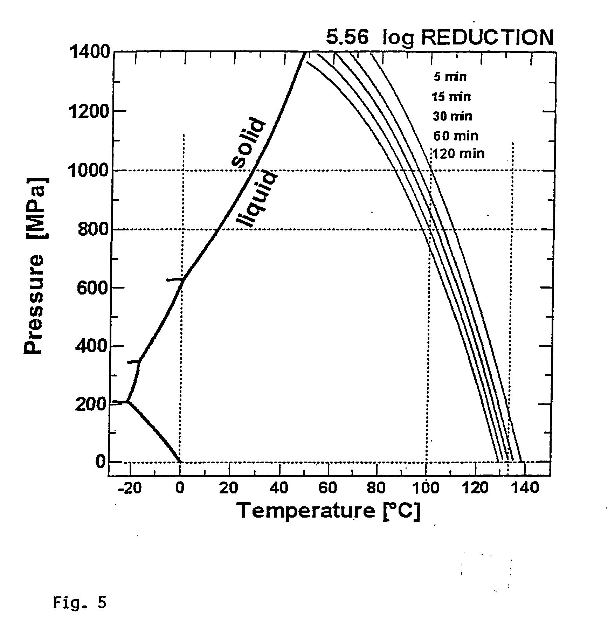

Bei diesen Temperaturen liegt bei feuchter Hitze ein Dampfdruck von ca. 0,4 MPa vor. Setzt man diese Bedingungen in die obige Gleichung für die auf LIA-Basis ermittelte Prioneninaktivierung ein, so ergeben sich 5,42 bzw. 5,67, im Mittel also 5,56 Zehnerpotenzen Reduktion. Für diese als ausreichend angesehene Reduktion können nun aus der oben entwickelten Gleichung für jeweils konstante Behandlungszeiten bestimmte Druck-Temperatur-Bedingungen ermittelt werden, die zu identischen Prioneninaktivierungen führen (siehe auch Fig. 5).
Ausgebend von den in der Literatur angegebenen Behandlungsbedingungen für die Hitzeinaktivierung bei Umgebungsdruck (p=0,1 MPa) zeigt sich auf Basis des vorhandenen Datenmaterials, daß durch Druckerhöhung ein identischer Effekt auf niedrigerem Eingangstemperaturniveau erreichbar ist.
Die erforderliche Behandlungstemperatur wird unter Berücksichtigung der entstehenden Kompressionswärme erreicht. Zeitgleich mit der Druckreduzierung sinkt das Temperaturniveau nach Abschluss der Druckbehandlung wieder auf den Ausgangswert zurück.
Beim erfindungsgemäßen Verfahren wird also zunächst die Art bzw. die Eigenschaften des zu behandelnden Produkts oder Gegenstands bestimmt. Wenn hier ein Produkt vorliegt, das z.B. ein Rohprodukt ist, dessen Eigenschaften durch die Behandlung nicht nachteilig beeinflußt werden sollen, dann wird unter Berücksichtigung der vorstehenden Beziehungen überprüft, bei welchem Druckwert auf niedrigerem Temperaturniveau der gewünschte Inaktivierungserfolg eintritt. Die Druck- bzw. Druck-Temperatur-Behandlung erfolgt dann in einer feuchten Umgebung, d.h. unter Nutzung einer reaktiven Flüssigkeit.
Eine weitere Größe im Entscheidungssystem neben der anzustrebenden minimalen Temperatur in Abhängigkeit von den Eigenschaften der Produkte ist eine möglichst kurze bzw. nicht zu lange Behandlungsdauer, um ein Einordnen des Verfahrens in übliche Produktionszyklen ohne nennenswerte Verringerung der Effektivität zu ermöglichen. Bei Materialien bzw. Gegenständen besonderer Art, wie beispielsweise Schlachthausmessern, Operationsbestecken oder dergleichen kann selbstverständlich eine gleichzeitige Temperaturbehandlung auf höherem Temperaturniveau gewählt werden.

## Patentansprüche

1. Verfahren zur gezielten Veränderung der Proteinstruktur von Prionen PrP[SC] oder infektiösen PrP[SC]-Ketten in tierischen, zum Verzehr oder zur Weiterverarbeitung vorgesehenen Rohprodukten, insbesondere Fleisch, Unterhäuten, Bindegewebe, Hirn und Knochen und/oder zur Sterilisation von Schlachthausgerätschaften, Operationsbestecken oder dergleichen mit infektiösen PrP[SC] kontaminierten Gegenständen, wobei die Rohprodukte und/oder Gerätschaften über ein Übertragungsmedium in einer feuchten Umgebung einer intensiven, von allen Seiten wirkenden Hochdruckbehandlung die eine oder mehrere Kompressions- bzw. Dekompressionsphasen beinhaltet unterzogen werden,

**dadurch gekennzeichnet, dass**

die Hochdruckbehandlung kombiniert mit einer Temperaturbehandlung auf niedrigem Niveau erfolgt und bei der Druckbehandlung die Kompressionswärme für die erforderliche Temperaturerhöhung des Behandlungsgutes genutzt wird sowie die zur Behandlung erforderlichen Druck-Temperatur-Bedingungen bei vorgegebener Reduktionszahl aus nachstehender Gleichung ermittelt werden:

$$\ln(k) = 86{,}05 - 0{,}728 \cdot T + 0{,}00133 \cdot T^2 + \left(\frac{36{,}79}{R \cdot T}\right) \cdot p$$

mit:

$C_t/C_0$: **Reduktion der Prionenkonzentration** $= (1 + k \cdot t \cdot (n-1))\frac{1}{1-n}$

k: Geschwindigkeitskonstante [1/min]
R: molare Gaskonstante: 8,314 J/(mol K)
p: Druck [MPa]
T: Temperatur [K]

## Claims

1. A method for the targeted modification of the protein structure of prions PrP$^{SC}$ or infectious PrP$^{SC}$ chains in animal raw products intended for consumption or for further processing, in particular meat, subcutaneous tissue, connective tissue, brain, and bones, and/or for the sterilisation of slaughterhouse equipment, surgical case or similar objects which are contaminated with infectious PrP$^{SC}$, with the raw products and/or equipment being subjected to an intensive high-pressure treatment acting from all sides via a transfer medium in a moist environment, which involves one or several compression and decompression phases, respectively,

   **characterised in that**

   the high-pressure treatment is performed combined with a heat treatment at a low level and the compression heat in the pressure treatment is utilized for the required temperature increase of the goods to be treated, and the pressure/temperature conditions required for the treatment with a pregiven reduction number are determined from the following equation:

$$\ln(k) = 86.05 - 0.728 \cdot T + 0.00133 \cdot T^2 + \left(\frac{36{,}79}{R \cdot T}\right) \cdot p$$

with:

$C_t/C_0$: $C_t/C_0$: reduction of the prion concentration $= \left(1 + k \cdot t \cdot (n-1)\right)\frac{1}{1-n}$

k: velocity constant [1/min]
R: molar gas constant: 8.314 J/(mol K)
p: pressure [MPa]
T: temperature [K]

## Revendications

1. Procédé pour la modification ciblée de la structure protéique de prions PrP$^{SC}$ et ou de chaînes infectieuses PrP$^{SC}$ dans des produits animaux bruts prévus pour la consommation ou pour la transformation, en particulier viande, épiderme, tissu conjonctif, cervelle et os, et/ou pour la stérilisation d'appareillages dans des abattoirs, d'instruments opératoires ou d'objets similaires contaminés avec du PrP$^{SC}$ infectieux, dans lequel les produits bruts et/ou les appareils sont soumis à un traitement intensif à haute pression agissant depuis tous les côtés via un fluide de transmission dans un environnement humide, traitement qui inclut une ou plusieurs phases de compression ou de décompression,

**caractérisé en ce que**

le traitement à haute pression a lieu de manière combinée avec un traitement en température à un faible niveau et, lors du traitement en pression, la chaleur de compression est utilisée pour l'augmentation de température nécessaire du produit à traiter, et les conditions de pression/température nécessaires pour le traitement sont déterminées à partir de l'équation suivante, pour un nombre de réduction prédéterminé :

$$\ln(k) = 86{,}05 - 0{,}728 \cdot T + 0{,}00133 \cdot T^2 + (36{,}79/R \cdot T) \cdot p$$

dans laquelle :

Ct/Co est la réduction de la concentration en prions =(1+k·t·(n-1))1/1-n
k est une constante de vitesse [1/minute]
R est une constante de gaz molaire = 8,314 J/(mol K)
p est la pression [MPa], et
T est la température [K].

Fig. 1

Fig. 2

Fig. 3

Fig. 4

Fig. 5